# EUROPEAN PATENT APPLICATION

(11) **EP 1 557 084 A2**
(43) Date of publication of application: **27.07.2005**
(21) Application number: 03757653.5
(22) Date of filing: 20.10.2003
(51) Int. Cl.: A01K 67/027

(54) **METHOD OF PRODUCING RECOMBINANT PROTEINS IN THE MAMMARY GLAND OF NON-TRANSGENIC MAMMALS**

(30) Priority: 21.10.2002 CU 23502
(71) Applicant: CENTRO DE INGENIERIA GENETICA Y BIOTECNOLOGIA, Ciudad Habana 12100 (CU)
(72) Inventor: TOLEDO ALONSO, Jorge, Roberto, 10300 Ciudad de La Habana (CU); SANCHEZ RAMOS, Oliberto, 10300 Ciudad de La Habana (CU); RODRIGUEZ MOLTO, Maria, Pilar, 10600 Ciudad de la Habana (CU); CASTRO REBOREDO, Fidel, Ovidio, 10600 Ciudad de la Habana (CU)
(74) Representative: Winckels, J.H.F., Mr. Ir.
(86) International application number: PCT/CU2003/000011
(87) International publication number: WO 2004/034780

(57) **Abstract**

The present invention relates to a method that allows for the production of bio-pharmaceutical proteins from the mammary gland of a non transgenic animal. The genes coding for such proteins are transferred to the mammary epithelial cells using adenoviral vectors. Said vectors can be based on replicative-deficient adenovirus, like ΔE1ΔE3, or on helper-dependant adenoviruses from which all or almost all regulatory sequences have been eliminated. By means of the method described in the present invention the production, it is possible to produce high levels of recombinant protein in the milk allowing for the large-scale production of bio-pharmaceutical proteins in their biologically active form.

## Description

### Description of the invention

The present invention relates to a method for the production of biologically active proteins of pharmaceutical value using the mammary gland as a bioreactor and recombinant adenoviruses as vectors for gene transfer.

The treatment of many diseases requires administration of bioactive proteins. These pharmaceuticals can be purified from blood or tissues in processes that in addition to being long and expensive carry the risk of transmission of infectious agents, such as those causative of AIDS and hepatitis.

The development of DNA-recombinant technology has allowed to the relatively cheap use of bacteria and yeasts as host systems for the production of pharmaceutically valuable proteins. Nevertheless in many occasions the biological activity of these proteins is affected due to the inefficient post-translational processing of these systems. For this reason, the production of a great number of biologically active proteins requires the biosynthetic machinery of higher eukaryotes. In this sense, systems based on baculovirus and mammalian cells have happened in viable strategies. Nevertheless the fermentation of animal cells is an expensive and technically demanding process.

The use of the mammary gland of transgenic animals as bioreactors has been identified as potential solution to the above mentioned problems. The main advantage of transgenesis relies on the possibility of creation of a new transgenic population from the founder animal, by natural reproduction. In spite of the promising perspective of this technology, limitations exist at the present time that prevents its use in a more generalized form. Particularly, when transgenic animals are generated in large farm animals, lot of time and effort is required for the production of a very limited number of G0, transgenic generation. Only a small proportion of transgenic G0s will express in the milk the protein of interest at reasonable levels as to allow the entrance in a commercial phase. An additional long time is required to multiply by means of crossing the G0 lines with high levels of expression, until reaching a suitable production capacity that allows the entrance in the market. During this scale-up period, there is also the risk that transgenic progeny will not express the exogenous protein at the levels of the original founder Somatic cloning as an alternative pathway allows overcoming some of the above mentioned limitations, however, the high cost as well as the inefficiency of this technique attempts against a more routine and wide application of it.

The use of transgenic mammals as biofactories implies, the construction of complex expression cassettes, in which the exogenous gene is linked to regulatory sequences allowing expression only in the mammary epithelial glandular tissue during lactation. In many occasions these regulating sequences do not work efficiently, causing that small amounts of recombinant protein are synthesized in other tissues, with a detrimental effect for the health of the animal.

Direct transfer of genes to the mammary glandular epithelium (MGE) in adult animals is a very promising strategy. This technology could not only diminish the production cost of bio-pharmaceutical drugs, but also reduce considerably the necessary time for the obtaining of these. The in vivo transformation of the MGE can be carried out in any animal, independently of its genetic background and with a minimum of technical requirements.

In an early work Archer et al (Proc Natl Acad Sci U S A. 1994, 91(15):6840-4) evaluated the feasibility of using retroviral vectors to transfer the gene of the human growth hormone (hGH) to the mammary epithelial cells of goats. hGH could be detected in milk although at very low concentrations, and this expression fell to basal levels after the first day.

In the patent documents US 5,215,904 and WO99/43795 also methods are described where authors used retrovirus vectors for the transformation of the MGE. Receptor-mediated endocytosis in EP 725141,A4 and the use of complexes based on polications and/or lipids in US 5.780.009 also have been described as alternatives for introduction of genes in the MGE. Although these works allow obtaining the protein of interest in the milk of the animals treated, until the moment the reported efficiencies are very low and the obtained protein levels is in the nanograms per milliliter range, what is serious constraint for the method to become useful in a productive process.

Adenoviral vectors can infect a wide range of cell types independently if they are actively dividing or not. In general terms, the expression of transgenes contained in adenoviral vectors is very robust. Upon infection, the virus remains episomal and therefore, expression is not affected by position effects. However, the expression of transgenes from adenoviral vectors is lost alter approximately 10 days. This is due mainly to a high immune response raised by the organism against expressed viral proteins.

In order to obtain a more prolonged expression of a gene, new adenoviral vectors have been developed from which most or all of the viral coding sequences have been removed (Robin J. Parks et al., Proc Natl Acad Sci U S A. 1996, 93(24):13565-70). Amplification of these vectors is achieved by coinfection of the producing cells with a second virus, which provides in "trans" all the required adenoviral proteins. This new generation of vectors has been named Helper-dependent or Gutless and they retain only the elements required for replication and packaging, the inverted terminal repeats (ITR) and the packaging sequences respectively. The cloning capacity of these vectors is of up to 36 Kb and has shown to be very stable in in vivo gene therapy assays.

The ability of adenoviral vectors to transfer genes in vivo to mammary epithelial cells of mice was shown by Yang and coworkers (Cancer Lett. 1995, 98(1):9-17). Since then to date several works have been published on the use of adenovirus to transfer genes to the MGE but in non of these works, the gene of interest was targeted for expression in the milk.

The possibility of using adenovirus as tools for the production of bioactive compounds is disclosed in the patent application WO97/17090. Nevertheless, although in this document the possibility of transformation of MGE is mentioned, all the experimental data showed as well as the results, are focused on the in vitro transformation of tissue culture samples, none of which was composed of mammary epithelial cells.

The only materialization of the invention that is revealed in WO97/17090 that describes the infection with adenovirus of the EGM, implies the injection of recombinant adenovirus in the mammary gland of lactating cows; nevertheless, according to this method, the levels of infection and therefore of recombinant protein production are considerably low and therefore does not provide evidences in support that transformation of MGE with adenoviral vectors can constitute a viable alternative for the production of biologically active pharmaceuticals in milk.

### Detailed description of the invention:

This invention relates to a method that allows the production of heterologous proteins at high levels in the milk of non-transgenic mammals. More specifically, this invention refers to the use of adenoviral vectors of type ΔE1ΔE3 helper-dependents to transfer foreign genes to the cells of the MGE. Cells transformed in this way synthesize and secrete biologically active proteins to the milk.

Therefore this it is in the scope of this invention to provide a simple and efficient means for the production of large quantities of complex biologically active proteins in the milk of non-transgenic mammals.

The method proposed herein allows for the efficient transformation in vivo of the mammary glandular epithelial cells. Said cells will have access to all the required genetic material, so that they can secrete biopharmaceutical proteins to the milk of treated animals. Specifically, in the present invention, adenoviral vectors are used to transfer foreign DNA inside cells of the MGE. Upon infection, viral DNA remains episomally in the nucleus of the infected cells, allowing expression of the transgene contained in the viral vector.

The method of the present invention implies the instillation of a solution containing adenoviral vectors directly through the channel of the teats. Animals should be preferably ruminants (e.g. bovine, ovine, or caprine). Animals might be subjected to a hormonal induction of mammogenesis and lactogenesis, alternatively, naturally lactating animals might be used. After infection, transgenes are transferred inside the mammary secretory cells, transcribed, translated and subjected to post-translational processes required before being secreted to milk. By means of milking, said tranfected animals become a living factory for the production of foreign proteins of biopharmaceutical interest.

Viral vectors should contain an expression cassette including DNA coding for the protein of interest, a signal peptide sequence for efficient secretion, this signal peptide might be from the coding gene or heterologous, a promoter sequence, not necessarily specific to the mammary glandular epithelium, a cleavage sequence and a poliadenilation signal.

Viral vectors are based preferably on human adenoviruses of types 2 and 5. Alternatively, adenoviral vectors might be used, lacking genes E1 and E3, or even lacking all the adenoviral coding sequences (helper-dependant or gutless)

The use of adenoviral vectors with defective replication (e.g. ΔE1ΔE3) as described in the present invention, allows for the expression of high levels of recombinant proteins in the milk, after approximately 10 days in imunocompetent animals. Said vectors are especially suitable for proteins required in small quantities for physic and chemical characterization or therapeutic treatments. This is the case for erythropoietin and tissue plasminogen activator.

Alternatively, as described in the method proposed, helper-dependant adenoviruses are the best option when large quantities of proteins are required. These vectors have shown to be very stable in the MGE, allowing for the expression in the milk of recombinant proteins for as long as five months in immunocompetent animals. Stability of gutless vectors can be improved depending on the immunogenicity of the recombinant protein or by using immunosuppressed animals. Gutless vectors in addition have a bigger cloning capacity (up to 36 Kb) allowing for the insertion of multiple expression cassettes.

### Expression cassettes

The adenoviral vectors of the present invention contain an expression cassette made out of a promoter, not necessarily specific for the MGE, a coding sequence for the gene of interest, a cleaving and polyadenilation sequence.

For construction of said expression cassette, constitutive promoters can be used; said promoters might be heterologous for the transformed cells. Examples of said promoters include early immediate human citomegaloviurs, actin, early SV40, myosin, and Roux Sarcoma Virus. Promoters naturally driving expression of proteins to the mammary gland might be of outstanding interest. Examples of such promoters include αS1 casein, αS2 casein, β lactoglobulin, κ-casein, whey acidic protein and α-lactalbumin.

The coding sequence may be a complementary DNA (cDNA) including or not an intron for the enhancement of the expression levels. Alternatively and depending on the cloning capacity of the vector, genomic DNA including introns from the gene can be used. This construct is preferred since better expression levels are obtained when compared with cDNA driven expression.

Virtually any heterologous protein can be expressed in the milk using the system proposed here. Particularly useful could be proteins with therapeutic or prophylactic value in humans or animals. Examples of proteins that can be obtained using this invention include, but are not limited to antigens (e.g. hepatitis B surface antigen) growth factors (e.g. human growth hormone, epidermal growth factor, insulin like growth factor, colony stimulating factor, granulocyte macrophage stimulating factor, nerve growth factor, erythropoietin) antibodies, cytokines (e.g. interleukin 6 or interleukin 2), α-1 antitrypsin, human serum albumin, β globin, tissue plasminogen activator, tumor suppressor proteins (e.g. p53), protein C and interferons.

Each of this heterologous protein produced according to this invention must be linked to a signal peptide directing secretion into the milk. Said signal peptide may be from the heterologous protein, if the protein is naturally secreted. When the protein is not secretable, the gene construct should include a heterologous signal peptide enabling secretion of the said protein.

Stability of messenger RNA is governed to a great extent by the region located in the 3' end of the gene. This region should include a cleaving and polyadenilation sequence. These sequences can be heterologous, but more commonly are used those derived from the globin gene, bovine growth hormone, Herpes Simplex tymidine kinase or early region of the SV40 virus.

### Adenoviral vectors

It has been shown that adenoviral vectors of human origin are capable to infect efficiently mammary epithelial cells of ruminant species, their stability in the secretory epithelium depends to a great extent on the degree of immunogenicity of the proteins expressed from the viral genome, this includes both proteins of viral origin and the biopharmaceutical protein. Replication-defective adenoviral vectors (e.g. ΔE1ΔE3) are capable of promoting high levels of expression of recombinant proteins, but have a reduced stability due to the strong immune response induced by viral proteins against infected cells.

Helper-dependent or gutless vectors do not contain in their genomes viral genes, hence the immune response against infected cells is much less and it is determined only by the immunogenicity of the recombinant protein itself. We have shown that these vectors are very stable and allow for the expression of biopharmaceutical protein in the milk of treated animals.

Replicative-defective adenoviral vectors can be constructed according to conventional techniques reported in literature (Tong-Chuan He et al., Proc. Natl. Acad. Sci USA 1998, 95(5):2509-14). Both packaging and amplification of the viral particles is carried out in HEK-293 cells or in any other cell line capable of complement the deficiency of the adenovirus. Helper-dependant adenoviral vectors can be constructed according to Robin J. Parks et al (Proc. Natl. Acad. Sci. USA 1996, 93(24):13565-70. For this, coinfection with a helper adenovirus is required, such helper virus should provide in trans all the proteins required for the correct packaging of the vector. In both cases, vectors are extracted from cells by three rounds of freezing and thawing. The functionality of the expression cassette contained in the adenoviral vectors can be assayed in vitro by the infection of cultured epithelial mammary cells. Depending on the infection, the protein of interest can be detected in the culture medium. Particularly useful in this regard, could be mammary epithelial cells HC11 or KIM2 of mouse origin and MAC-T of bovine origin. It is advisable to purify viral particles by double centrifugation in a CsCl gradient in order to eliminate the cellular debris and defective particles that could potentially interfere with the efficient infection of the mammary epithelial cells. For purification of helper-dependant adenoviral vectors, centrifugation in a CsCl gradient is mandatory for cleaning up possible contaminations with the helper adenovirus. Adenoviruses purified by this method are dialyzed because the high concentration of CsCl could interfere with the viral infection of cells and tissues. Once dialyzed, adenoviruses can be stored at -80 degrees Celsius, without noticeable changes in their titer.

### Animals

Present invention can be applied to all mammals; nevertheless, ruminants are preferred due to their higher milk production capacity. Animals in the early stages of sexual maturation can be used. These animals are subjected to a hormonal regime in order to induce mammogenesis and lactation. As a general rule, naturally lactating animals have higher levels of milk production and therefore of the transgene than animals with hormonally induced lactation

### Infusion

### Infusión

Before infusion with the solution containing the adenoviral vectors, the animal must be milked, to eliminate the milk container in the cisterns. Mammary gland is then infused with an isosmotic solution via the channel of the teat, until the gland becomes full, by massaging, of the gland, the solution will reach the totality of the alveoli, and the solution is removed by milking. This step is to be repeated two to three times to assure the total rinsing of the mammary gland and distention of the alveoli and of the ductal tissues. The isosmotic solution can be PBS, NaCl 0.9%, glucose 5%, HBS tissue culture medium.

Infusion of the solution containing the viral particles is conducted directly through the channel of the teat. As carrying liquid, isotonic solutions such as glucose 5%, PBS, NaCl 0.9%, HBS, or tissue culture medium (e.g.. DMEM) can be use. The viral concentration in the infusion solution can be variable, although concentration of 1 x 10⁹ PFU/ml or higher are desired. Optimal volume of infusion may vary depending on the size of the mammary gland, for goats for instances, volumes can vary between 50 and 300 ml per mammary gland.

To make the infusion through the channel of the teat, a syringe coupled to a canula can be used. Infusion should be performed slowly, and massaging is required during and after it in order to assure a homogenous distribution of the solution in all the mammary epithelial cells.

### Collection of the heterologous protein

Milk from treated animals, can be obtained by conventional methods of hand or automated milking. Caseins and fat are separated from whey, most part of which is stored at -20° C, while small fractions are used to detect and quantify the protein of interest, by means of commonly known analytical techniques (e.g. ELISA, Western blotting, and biological activity). Whey containing considerable quantities of the interest proteins are mixed and used as active row material for the purification of the heterologous protein. The purification process can vary considerably depending on the given protein.

In this way, the method for the production of high levels of a recombinant protein in the milk according to the present invention requires the following steps:
1. Construction of a recombinant adenovirus containing an expression cassette with the desired gene. This step involves:
   a)- construction of the recombinant adenoviral genome
   b)- production of viral particles in the cell line 293
   c)- amplification and purification of adenoviral vectors
2. Infection of the mammary epithelial cells. This step involves:
   a)- induction of mammogenesis and of lactation in the case that an animal with no natural lactation is to be used.
   b)- infusion of the mammary gland with a solution containing the viral particles
3. Recover of the recombinant protein. This step involves:
   a)- milking of the treated animal
   b)- detection and quantitation of the heterologous protein in collected milk
   c)- purification of the heterologous protein.
Some illustrative examples follow for a better understanding of the present invention:

### Examples

### Example 1. Construction of adenoviral vectors (ΔE1ΔE3) containing the genes for hGH and hEPO.

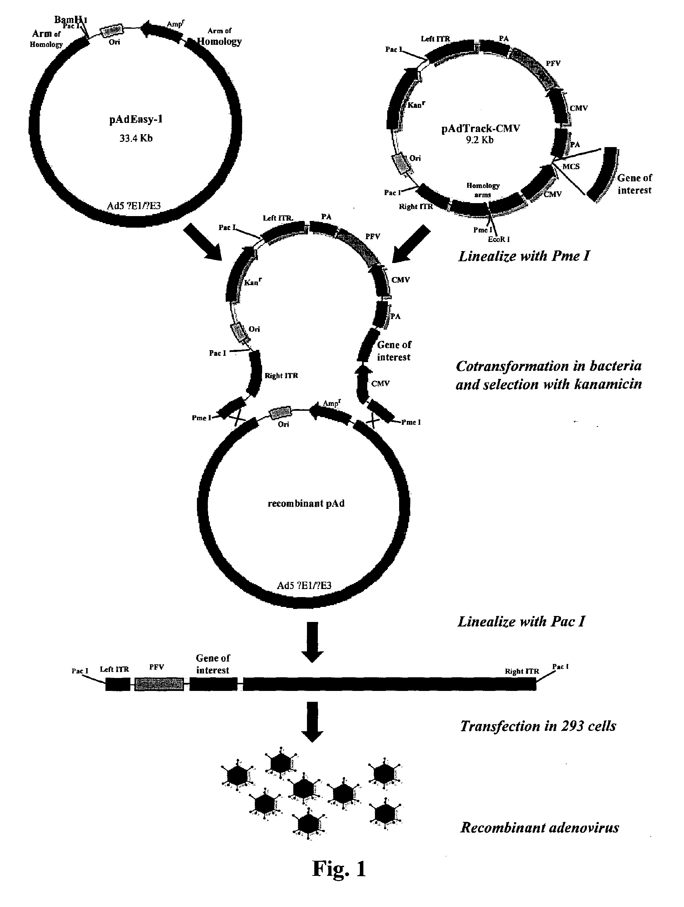

Adenoviral vectors with defective replication were constructed based on the Adeasy system (Tong-Chuan He et al.; Proc Natl Acad Sci U S A. 1998, 95(5):2509-14), as transfer vector, the plasmid pAdTrack-CMV (Fig. 1) was used. The system based on AdEasy constitutes a fast and easy option for making recombinant adenoviruses. A two step process is required where first the expression cassette is
cloned into a transfer vector and subsequently transfer to the viral genome by homologous recombination in the bacterial strain BJ5183. The adenoviral genome is then digested with Pac I endonuclease and transfected into 293 or 911 cell line. In our case, both the formation of infective virions as well as the ultimate amplification was monitored by co expression of green fluorescent protein (GFP) contained in the transfer vector pAdTrack-CMV.

### Example 2: Construction of helper-dependent adenoviral vectors

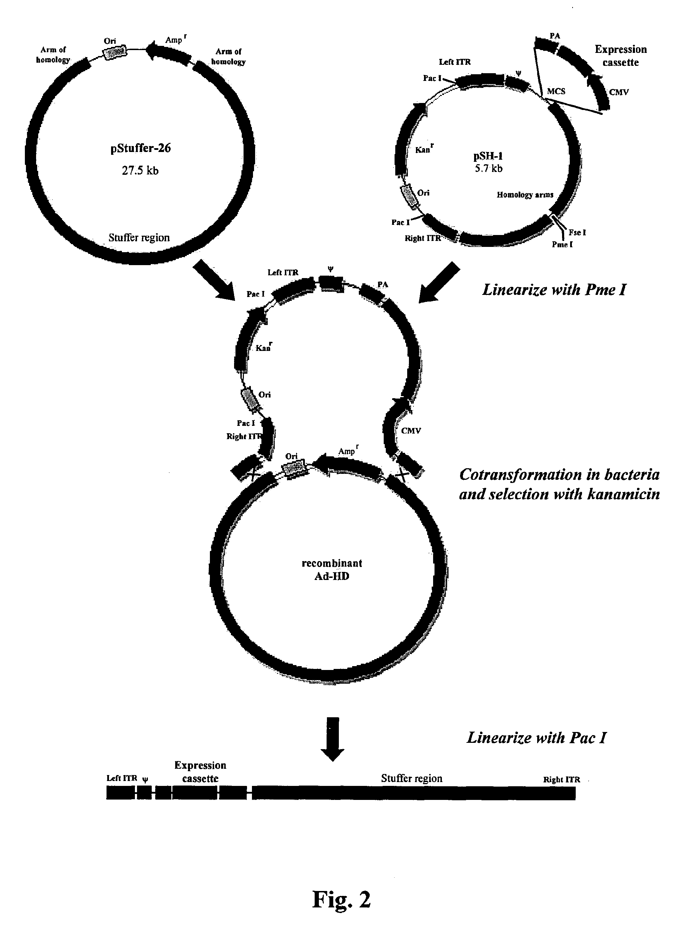

Helper-dependent adenoviral vectors were constructed by cloning an expression cassette containing the gene of erythropoietin in the multi cloning site of plasmid pSH-1. With the goal of giving to the resultant plasmid an appropriate size allowing for packaging, it was transferred to the pStuffer-26 vector, by homologous recombination in the bacterial strain BJ5183 (Fig. 2). As a result of the homologous recombination a plasmid of more than 28 Kb was generated. Upon digestion with Pac I endonuclease the plasmid is transfected into the 293-Cre cell line and later infected with the helper adenovirus AdInvLψL-GFP. The expression of Cre recombinase in the 293-Cre cell line promotes the recombination between Lox P sites flanking the packaging sequence in the helper adenovirus, which will lose the capacity of being packaged, but will keep the ability to replicate and therefore to provide in trans the viral proteins required for the formation of viral particles containing the genome of the helper-dependent adenoviral vector.

### Example 3: Expression of human growth hormone (hGH) and human erythropoietin (hEPO) from adenoviral infected primary mammary epithelial cultured cells

In order to test the correct functioning of the expression cassettes for hGH and hEPO constructed in the adenoviral vectors, mammary epithelial HC11 cells were infected. Cells were cultured in DMEM supplemented with EGF (10ηg/ml) and insulin (10 µg/ml). At 80 % of confluence, adenoviral vectors at a ratio of 20 particles per cell were added to the cell culture. Twenty four hours later, the medium was changed to the same medium, but supplemented with EGF and insulin, but without FCS. After 48 hours, the medium was collected, and the proteins contained in 1 ml were precipitated with trichloroacetic acid and resuspended in 40 µl of water, 20 µl were used for electrophoresis and subsequent Western blotting assay.

### Example 4: Expression of human growth hormone (hGH) and human erythropoietin (hEPO) in the milk of mice

The ability of adenoviruses to infect MGE and to further promote secretion to the milk of the protein of interest was assayed in mice. Three experimental groups were made with 5 mice in each. B6D2F1 female mice at day 17 of gestation were infused with 100 µl of a preparation containing virus depending on the experimental group: I-2.5x10⁷ GTU/ml of adenovirus (ΔE1ΔE3) containing the gene of hGH; II- 1x10⁹ GTU/ml of adenovirus (ΔE1ΔE3) containing the gene of hEPO; III- saline solution.
Milking of treated mice began after the second day post-partum. Collected milk was diluted 1:5 in separation buffer (10 mM Tris-HCl pH 8, 10 mM CaCl₂) and the caseins were separated from whey by cold centrifugation at 4° C for 30 minutes at 15 000 g.

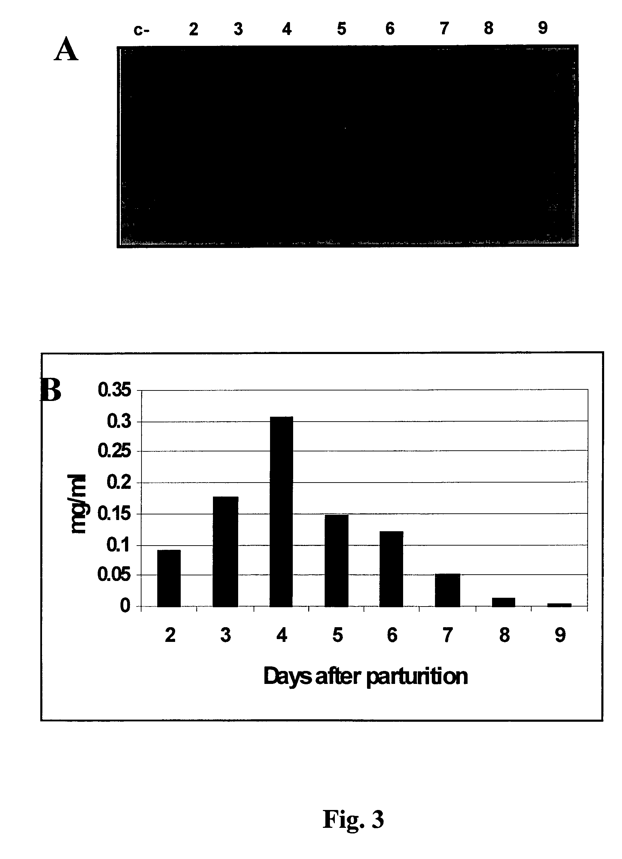

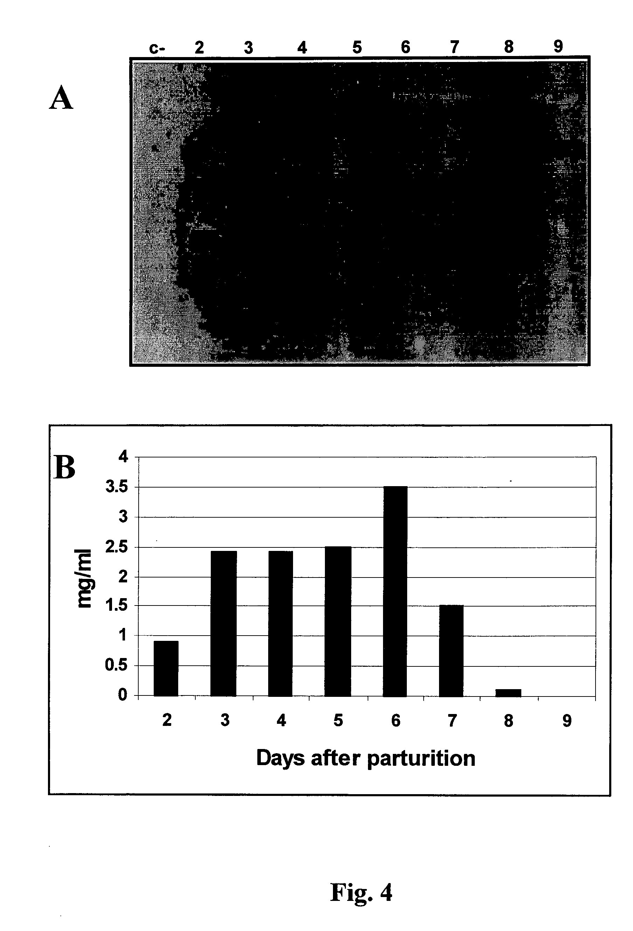

The content of hGH in milk simples was detected by Western blotting and quantified by ELISA (hGH ELISA, Boehringer Mannheim, Cat. No. 1 585 878) (Fig. 3). The content of hEPO was also detected by Western blotting and quantified by ELISA (ELISA manufactured at the CIGB) (Fig. 4).

### Example 5: Induction of lactation in goats.

Recombinant adenoviral vectors for the transfer of genes to the mammary gland can be used virtually in any species of mammals; however ruminants are preferred due to their high milk production capacity. Animals in their early stages of sexual maturation can be used to which mammogenesis and lactogenesis can be hormonally induced. Animals in natural phase of lactation can be used as well. If the animals to be used are goats for example, hormonal induction can be made by the administration of
estradiol (0.25 mg/kg, i.m) and progesterone (0.75 mg/kg, i.m.) on days 1, 3, 5, 7, 9, 11 y 13 while prednisolone (0.4 mg/kg, i.m) should be administered on days 14 to 16 with daily massages on the udder starting on day 5.

### Example 6: Infusion of a recombinant adenovirus (ΔE1ΔE3) containing the gene of the human growth hormone hGH in the mammary gland of goats.

Goats in the phase of lactation were given intramuscularly with 10 mg of diazepam in order to diminish the stress during the treatment. Animals were milked extensively to eliminate as much milk as possible from the cisterns; mammary glands were rinsed twice by the infusion of 200 ml saline at 37° C and subsequently milked. All the infusions were made directly through the channel of the teat with a 50-ml syringe coupled to a catheter. Infusions were performed slowly while simultaneously massaging the infused udders.
In goats, udder is separated in two independent halves; one of those was infused with a solution containing the adenoviral vectors, while the other received only saline and was used as a negative internal control. To each mammary gland a solution of 200 ml of saline containing a viral load of 10⁹ GTU/ml, what means, each mammary gland received in total 2 x 10¹¹ viral particles. After infusion, udder was massaged to facilitate homogeneous distribution of the solution and to allow it to reach all the ducts and alveoli. The infused solution was removed on the next day by milking.

### Example 7: Infusion of a recombinant helper-dependent adenovirus containing the gene of erythropoietin in the mammary gland of goats.

Goats in the phase of lactation were given intramuscularly with 10 mg of diazepam in order to diminish the stress during the treatment. Animals were milked extensively to eliminate as much milk as possible from the cisterns; mammary glands were rinsed twice by the infusion of 200 ml saline at 37° C and subsequently milked. All the infusions were made directly through the channel of the teat with a 50-ml syringe coupled to a catheter. Infusions were performed slowly while simultaneously massaging the infused udders.
In goats, udder is separated in two independent halves; one of those was infused with a solution containing the adenoviral vectors, while the other received only saline and was used as a negative internal control. To each mammary gland a solution of 200 ml of saline containing a viral load of 10⁹ GTU/ml, what means, each mammary gland received in total 2 x 10¹¹ viral particles. After infusion, udder was massaged to facilitate homogeneous distribution of the solution and to allow it to reach all the ducts and alveoli. The infused solution was removed on the next day by milking.

### Example 8: Detection of hGH and hEPO in the milk of goats infused with adenoviral vectors

Milk from infused animals was collected by hand milking starting 48 hours post infusion. Two daily milking were performed, one in the morning and the other in the late afternoon. Most of the collected milk was stored at -70° C for ulterior purification of the protein, while small samples were used in order to detect and quantify the content of hGH and hEPO in each of the lots.

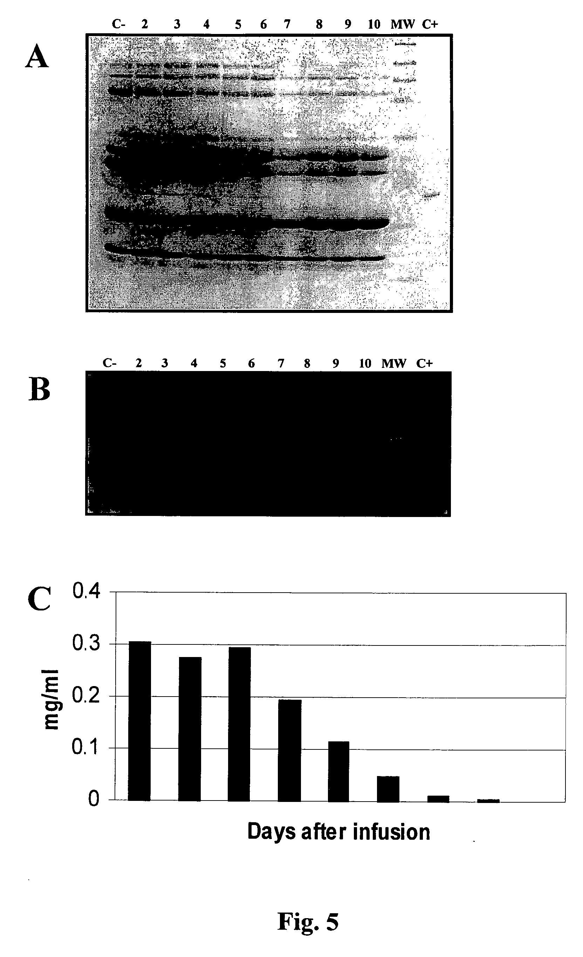

Detection of hGH or hEPO was carried out as follows. To 150 □1 milk simples, four volumes of separation buffer (10 mM Tris-HCl, 10 mM CaCl₂) were added and centrifuged at 4° C for 30 minutes at 15000 g to separate whey from fat and caseins. Whey fraction was recovered and the proteins contained in 10 µl were separated by SDS-PAGE on a 12.5%. acrilamide gel. Proteins were transferred to nitrocellulose filters and labeled with a polyclonal antibody against hGH (1/500). Immunoreactive bands were visualized by chemiluminiscent system (ECL) from Amersham Pharmacia Biotech (Fig. 5B).

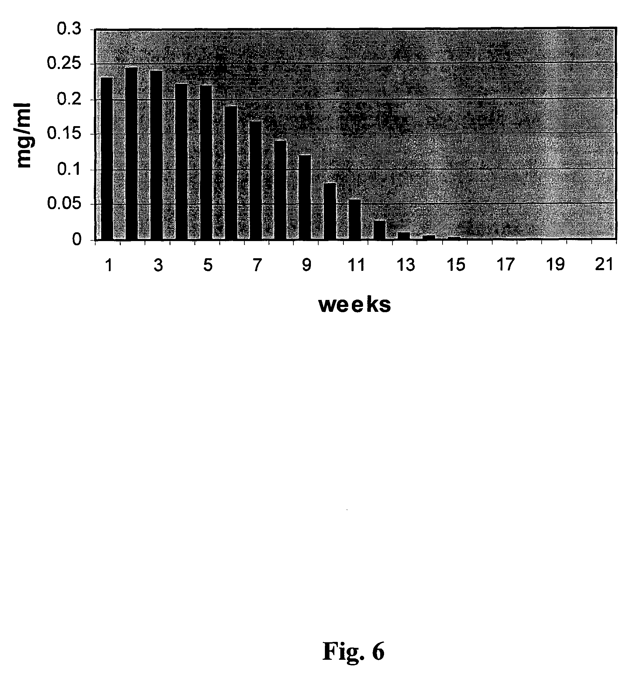

Quantitation of hGH was made by hGH ELISA from Boehringer Mannheim (Cat. No. 1 585 878). All the procedure was carried out according to manufacturer's instructions.
Quantitation of hEPO was made by a sandwich ELISA manufactured at the Center for Genetic Engineering and Biotechnology, Havana, Cuba.
In animals infused with adenoviral vectors of type ΔE1ΔE3, hGH was detected up to the day 11 after infusion reaching 0.3 mg/ml during the first three days. (Fig. 5C).
In the case of animals inoculated with helper-dependent adenoviral vectors, the levels of hEPO were also considerably high (Fig 6). The use of these vectors yielded expression levels superior to 0.2 mg/ml during the first 5 weeks, declining slightly until reaching 5ng/ml on day 145 post infusion

### Advantages of the proposed solution

The method proposed in the present invention constitutes a solution to the growing needs of production of biopharmaceuticals of protein constitution. Specifically, the present invention allows for large scale production of proteins whose biological activity is tightly linked to complex post translational processing and hence, depends on the biosynthetic machinery of higher organisms. The method proposed herein makes possible the production of such proteins in a fast, simple and economically achievable. In this sense, the present invention allows for a fast answer in a short period of time to the changes in the market, its application does not involve big technical requirements and the reaction capacity can be easily adjusted depending on the needs.

Additionally, the present invention allows for saving of time and of resources as an alternative for the study of post translational modifications of the mammary gland of different species. Taking into account that the coding sequences of the protein under study is contained in an adenoviral vector, this can be transferred to the mammary gland of a wide range of species, allowing in this way, the study of differential modifications that the same protein is subjected to depending on the species in which is produced.

### Brief description of the figures.

**Figure 1:** Shows the strategy used for the construction of adenoviral vectors ΔE1ΔE3 containing the genes of hGH y hEPO.
**Figure 2:** Shows the strategy used for the construction of the helper-dependent adenoviral genome containing the gene of hEPO.
**Figure 3:** Assay for the expression of hGH in the milk of mice infused with 2.5x106 GTU per mammary gland of an adenovirus ΔE1ΔE3 containing the gene of hGH under the control of a human cytomegalovirus promoter. A) Western blot assay: negative control (C-) refers to the milk of a mouse infused with an adenovirus lacking the expression cassette for hGH, lanes 2,3,4,5,6,7,8,9 contain milk samples collected at the correspondent lactation days. B) hGH concentration in the milk in relation with the days post partum
**Figure 4:** Assay for the expression of hEPO in the milk of mice infused with 1x10⁸ GTU per mammary gland of an adenovirus ΔE1ΔE3 containing the gene of the hEPO under the control of a human cytomegalovirus promoter. A) Western blot assay: negative control (C-) refers to the milk of a mouse infused with an adenovirus lacking the expression cassette for hEPO, lanes 2,3,4,5,6,7,8,9 contain milk samples collected at the correspondent lactation days. B) hEPO concentration in the milk in relation with the days post partum
**Figure 5:** Shows the presence of hGH in the milk of goats infused with 2x10¹¹ GTU per mammary gland o fan adenoviral vector ΔE1ΔE3 containing the gene of hGH under the control of a human cytomegalovirus promoter. A) Electrophoresis of milk samples collected in the first ten days alter infusion, negative control corresponds to the milk from a mammary gland infused with an adenoviral vector lacking the expression cassette for hGH. B) Western blot assay showing the expression of hGH in the milk of goats; negative control corresponds to the milk from a mammary gland infused with an adenoviral vector lacking the expression cassette for hGH; positive control corresponds to a recombinant hGH purified from bacterial cells. C) hGH concentration in the milk in relation with the days post infusion
**Figure 6:** Results of an ELISA showing the levels of hEPO in the milk of goats infused with a helper-dependent adenoviral vector containing the gene of the hEPO under the control of a human cytomegalovirus promoter.

## Claims

1. A method for the production of heterologous proteins in the milk of non human mammals mediated by the transformation of mammary glandular epithelial cells (MGE) with adenoviral vectors, comprising the following steps:
a) Induction of lactation of a mammals during the early stages of its sexual maturity
b) Removing of milk and exhaustive washing of the interior of the mammary gland prior viral infusion
c) Infusion through the channel of the teat, until completion of the capacity of the mammary gland, of a solution containing adenoviral vectors carrying genes coding for heterologous proteins of interest.
d) Drain up of the mammary gland between 4 and 24 hours post infusion
e) Collection of the milk contained in the mammary gland starting 48 hours alter infusion
f) Purification from the milk of heterologous proteins of interest.

2. A method as claimed in claim 1, in which the adenoviral vector contains the majority of adenoviral genes.

3. A method as claimed in claim 1, in which the adenoviral vector is an adenovirus lacking the majority or all the adenoviral genes and requires a helper adenovirus that provides in trans all the necessary proteins required for the formation of viral particles.

4. A method as claimed in claim 1, in which the heterologous proteins of interest are selected from a group consisting of growth factors such as human growth hormone, epidermal growth factor, insulin-like growth factor, granulocyte-macrophage colony stimulating factor, nerve growth factor, erythropoietin, coagulation factors such as FVIII and FIX, antibodies, cytokines such as interleukin-6 or interleukin-2, α1-antitripsina, human serum albumin, β-globins, tissue plasminogen activator, tumor suppressor proteins such as P53, protein C or interferons.
